# EUROPEAN PATENT SPECIFICATION

(11) **EP 1 803 471 B1**
(45) Date of publication and mention of the grant of the patent: **07.09.2011**
(21) Application number: 06025987.6
(22) Date of filing: 14.12.2006
(51) Int. Cl.: A61L 9/14, A61L 9/16, F24F 6/04

(54) **Air filtering apparatus**
Luftfilterungsvorrichtung
Appareil de filtrage de l'air

(30) Priority: 28.12.2005 JP 2005377511; 28.12.2005 JP 2005377602; 28.12.2005 JP 2005377603; 28.12.2005 JP 2005377733
(43) Date of publication of application: 04.07.2007
(73) Proprietor: SANYO ELECTRIC CO., LTD., Moriguchi-shi, Osaka (JP)
(72) Inventor: Fukushima, Toshio, Ota-shi,Gunma 373-0813 (JP); Arakawa, Toru, Tatebayashi-shi, Gunma 374-0066 (JP); Usui, Hiroaki, Oizumi-machi,Ora-gun, Gunma 370-0533 (JP); Tamura, Takaaki, Oizumi-machi, Ora-gun,Gunma 370-0533 (JP); Koyama, Koji, Ota-shi,Gunma 373-0815 (JP); Mizuma, Masato, Ota-shi,Gunma 373-0812 (JP); Uchida, Yoichi, Ashikaga-shi,Tochigi 326-0006 (JP); Takahashi, Kazuo, Ota-shi,Gunma 373-0036 (JP)
(74) Representative: Glawe, Delfs, Moll

(56) References cited:
- EP-A- 1 619 451
- JP-A- 2002 181 358
- JP-A- 2002 349 913

## Description

### 1. Field of the Invention

The present invention relates to a portable air filtering apparatus that can remove (inactivate, sterilize, etc.) microorganisms such as virus, bacteria, fungus, etc. floating in the air, for example, and particularly relates to a portable floor-mount type air filtering apparatus.

### 2. Description of the Related Art

For the purpose of removing microorganisms such as virus, bacteria, fungus, etc. floating in the air, there has been proposed a sterilizing apparatus in which electrolytic water mist is diffused into the air so that the electrolytic water mist is brought into direct contact with microorganisms floating in the air, thereby inactivating the microorganisms such as virus, bacterial, fungus, etc.

According to this sterilizing apparatus, since the electrolytic water mist is diffused into the air, water containing hypochlorous acid or the like is supplied and infiltrated into a humidifying element formed of non-woven cloth and air blown from an air blower is applied to the humidifying element to thereby discharge moisture of the humidifying element (for example, see JP-A-2002-181358).

The above-described sterilizing apparatus works effectively under a use environment which electrolytic water mist of mine particles easily reach, that is, in a relatively small space, however, it does not work effectively in a user environment which the electrolytic water mist hardly reaches, that is in a large space such as a kindergarten, an elementary/junior/high school, long-term care insurance facilities, a hospital or the like.

### SUMMARY OF THE INVENTION

Therefore, the present invention has been implemented in order to solve the above problem, and a first object of the present invention is to provide a floor-mount type air filtering apparatus that stably perform sterile filtration of air passing through a filter and further can perform sterile filtration of air in a large space.

A second object of the present invention is to provide a floor-mount type air filtering apparatus that can water-tightly secure a water receiving tray to a partition plate.

A third object of the present invention is to provide a floor-mount type air filtering apparatus in which a support tray and a circulating pump do not interfere with each other when the support tray is detached.

A fourth object of the present invention is to provide a floor-mount type air filtering apparatus that can secure the flow rate and amount of air blown from an air blower to a gas-liquid contact member.

A fifth object of the present invention is to provide a floor-mount type air filtering apparatus that can effectively cool a control board portion for controlling an electrolytic water generating unit, etc.

In order to attain the above objects, an air filtering apparatus (for example, a floor-mount type air filtering apparatus) according to the present invention comprises the features of claim 1.

According to the above-described floor-mount type air filtering apparatus, the inside of the housing of the air filtering apparatus is sectioned into plural chambers by at least one partition plate, and one chamber is used as an air filtering chamber for performing sterile filtration while the other chamber is used as an electric-component chamber for accommodating electric component parts or an electrolytic water supply chamber for accommodating the electrolytic water supply unit. Accordingly, the electrolytic water in the air filtering chamber is prevented from spattering into the electric component chamber and further the constituent elements of the floor-mount type air filtering apparatus can be accommodated in the housing in order and in a compact arrangement.

Furthermore, in the above-described floor-mount type air filtering apparatus, one end portion (9A) of the water receiving tray (9) may penetrate through one partition plate (51) so as to extend to the other chamber (61).

According to the above construction, the end portion of the water receiving tray penetrates through the partition plate so as to extend to the other chamber side, and thus the water receiving tray can be water-tightly secured to the partition plate without inducing any gap between the partition plate and the water receiving tray. Furthermore, the end portion of the water receiving tray is inserted through an opening portion formed in the partition plate to fix the water receiving tray to the partition plate, and thus the fixing work can be easily performed.

In the above-described floor-mount type air filtering apparatus, a cover member (72) for covering the end portion (9A) of the water receiving tray (9) may be provided in the other chamber (61).

Furthermore, antirust coating may be applied to the water receiving tray (9).

Still furthermore, a water return portion (back board) (71) may be provided to the end portion of the water receiving tray.

The water receiving tray (9) may be disposed so as to downwardly incline from the other chamber (61) to the one chamber (60).

Furthermore, the electrolytic water contains active oxygen species achieved by electrolyzing water such as tap water or the like, and the active oxygen specifies contain at least one of hypochlorous acid, ozone and hydrogen peroxide.

Still furthermore, in the above-described floor-mount type air filtering apparatus, the electrolytic water supply unit may contain a support tray (10) for receiving electrolytic water flowing from the water receiving tray (9) located below the gas-liquid contact member (5) and stocking the electrolytic water concerned, and a circulating pump (13) that is inserted in the support tray and pumps up the electrolytic water stocked in the support tray, wherein one of a projecting portion (50A) and a rail groove (53) in which the projecting portion (50A) is fitted so as to be movable along the rail groove (53) is provided to at least one of both the side surfaces (10c, 10d) of the support tray (10), the other of the projecting portion and the rail groove is provided to at least one of the inner side surface (2Z) of the housing and the side surface (52c) of the partition plate (52), the inner side surface (2Z) and the side surface (52c) facing the side surfaces (10c, 10d) of the support tray (10), and the rail groove (53) is designed so that the projecting portion (50A) is guided along the rail groove (53) and the support tray (10) is prevented from abutting against the circulating pump (13) when the support tray (10) is detached from the housing (2). In this case, the projecting portion (50A) may be downwardly guided along the rail groove (53) until the circulating pump (13) is located at the outside of the support tray (10).

Furthermore, the rail groove (53) may comprise a vertical movement portion (53b)and a horizontal movement portion (53a) intercommunicating with the vertical movement portion (53b), the support tray (10) being movable in one of a downward direction and an obliquely downward direction and horizontally movable along the horizontal movement portion (53a) and detached from one end of the horizontal movement portion (53a) to the outside of the housing (2) when the support tray (10) is detached from the housing (2) .

According to the above construction, by moving the projecting portion of the support tray along the rail groove, the support tray can be detached from the housing without interfering with the circulating pump.

Specifically, the rail groove comprises the horizontal movement portion extending substantially in the horizontal direction from the take-out side of the support tray to the back side of the housing, and the vertical movement portion extending from the back end of the horizontal movement portion upwardly or obliquely upwardly. Therefore, the support tray can be guided along the vertical movement portion downwardly or obliquely downwardly from the state that the support tray is secured to the housing, and further drawn out (taken out) along the horizontal movement portion.

Accordingly, the rail groove is formed such that the projecting portion is downwardly guided along the rail groove until the circulating pump is located at the outside of the support tray when the support tray is detached from the housing. Therefore, by moving the projecting portion of the support tray along the rail groove, the support tray can be detached from the housing while the support tray and the circulating pump do not interfere with each other.

Furthermore, the support tray can be guided downwardly or obliquely downwardly along the vertical movement portion from the state that the support tray is secured to the housing. Therefore, by moving the projecting portion of the support tray along the rail groove, the support tray can be detached from the housing while the interference between the support tray and the circulating pump can be more surely prevented. Still furthermore, the support tray can be drawn out to the take-out side along the horizontal movement portion, and thus the support tray can be horizontally drawn out without being inclined.

The above-described floor-mount type air filtering apparatus may be further equipped with an air guide plate for guiding air from an air blow-out port (7a) of the air blowing fan (7) to the gas-liquid contact member (5). The air guide plate may comprise an extension portion (50a) extending in a flow direction of air blown out from the air blowing fan (7), and a guide portion (50b) that is connected to the extension portion (50a) and extends obliquely from the blow-out port (7a) toward the gas-liquid contact member.

Furthermore, the floor-mount type air filtering apparatus may be further equipped with a support plate (8) for isolating the gas-liquid contact member and the air blowing fan from each other, and the support plate (8) is provided with an opening (8a) through which the air blown out from the air blowing fan is passed.

Still furthermore, The inclination angle of the guide portion (50b) is adjustable with respect to the extension portion (50a).

The length of the extension portion (50a) is adjustable.

According to the above construction, the air blown out from the air blowing fan is passed through the extension portion, so that the air can be guided to the guide portion without reducing the air flow rate and the air flow amount.

Furthermore, the gas-liquid contact member and the air blowing fan can be isolated from each other by the support plate, and the opening through which the air blown out from the air blowing fan is passed is formed in the support plate. Therefore, the mount side of the air blowing fan and the mount side of the gas-liquid contact member are isolated from each other by the support plate, so that the air blown out from the air blowing fan is prevented from returning to the air blowing fan side.

Still furthermore, the inclination angle of the guide portion is adjustable with respect to the extension portion, and thus the air flow direction can be varied in conformity with the shape and size of the gas-liquid contact member and the mount position of the gas-liquid contact member.

Still furthermore, the length of the extension portion is adjustable, and thus the length of the extension portion can be adjusted so that neither turbulent flow nor eddy occurs in the air stream from the air blowing fan.

Accordingly, the air blown out from the air blowing fan is temporarily guided by the extension portion of the air guide plate, whereby neither turbulent flow nor eddy occurs in the air stream, and thus the air flow rate and air flow amount of the air blown out from the air blowing fan can be kept with no change. Furthermore, indoor air can be fed from the blow-out port of the air blowing fan to the gas-liquid contact member located at a remote position by the guide portion, whereby the air can be uniformly applied onto the gas-liquid contact member.

Furthermore, by providing the support plate, the air of the air blowing fan can be prevented from escaping to the air blowing fan side. As a result, the air flow amount of the air fed to the gas-liquid contact member can be increased.

Still furthermore, by varying the inclination angle of the guide portion, the air flowing direction can be changed in conformity with the size and position of the gas-liquid contact member. Accordingly, air (for example, indoor air) can be evenly applied to the whole gas-liquid contact member.

Furthermore, by adjusting the length of the extension portion, occurrence of turbulent flow and eddy in air stream can be prevented. Accordingly, the air flow amount and the air flow rate can be prevented from being reduced.

In the above-described floor-mount type air filtering apparatus, the controller may contain a control board portion having electric parts and a heat sink for the electric parts, and the heat sink may be disposed so as to penetrate through an opening (51) formed in the partition plate (51) and face a suction port (3) of the air blowing fan (7).

Furthermore, the inside of the housing (2) may be vertically partitioned by one partition plate (51), the suction port (3) of the air blowing fan (7) may be substantially horizontally disposed, and air blown out from the air blowing fan to the gas-liquid contact member (5) is sucked from the suction port (3) that is formed in the housing and located at the lower side of the air blowing fan.

Furthermore, the electrolytic water supply unit may have electrodes for electrolyzing water to generate electrolytic water and the electric parts may contain a part for controlling electrodes of the electrolytic water supply unit.

Still furthermore, the electrolytic water generated in the electrolytic water supply unit may be electrolytic water containing active oxygen species achieved by supplying current to the electrodes and electrolyzing tap water.

Still furthermore, the active oxygen specifies may contain at least one of hypochlorous acid, ozone and hydrogen peroxide.

According to the above construction, the heat sink of the electrical parts of the control board portion penetrates through the opening formed in the partition plate, and is located so as to face the suction port of the air flowing fan. Therefore, indoor air flows while coming into contact with the heat sink, and then is sucked into the suction port of the air blowing fan. Accordingly, the cooling of the heat sink is promoted, and thus heat radiation of the electrical parts is also promoted. Furthermore, since the heat sink is disposed so as to penetrate through the opening of the partition plate, the heat sink may be inserted from the other chamber into the one chamber while the control board portion is secured to the heat sink in advance, and then secured to the partition plate by screws or the like. Therefore, the work of fixing the heat sink can be enhanced.

### BRIEF DESCRIPTION OF THE DRAWINGS

Fig. 1 is a perspective view showing an embodiment of a floor-mount type air filtering apparatus according to the present invention;
Figs. 2A and 2B are perspective views showing the internal construction of the floor-mount type air filtering apparatus;
Fig. 3 is a longitudinal-sectional view of a housing;
Fig. 4 is a front view showing a gas-liquid contact member;
Figs. 5A to 5C are systematic diagrams showing a mechanism for dropping electrolytic water to the gas-liquid contact member, wherein Fig. 5A is a side view of the mechanism, Fig. 5B is a cross-sectional view of a water spray portion (water spray box) and Fig. 5C is a diagram showing the construction of an electrolytic bath;
Fig. 6 is a perspective view showing the internal construction of the housing;
Fig. 7 is a perspective view showing a water receiving tray penetrating through a partition plate which is viewed from an electric component chamber;
Fig. 8 is an exploded perspective view showing a state that the partition plate and the water receiving tray are assembled with each other;

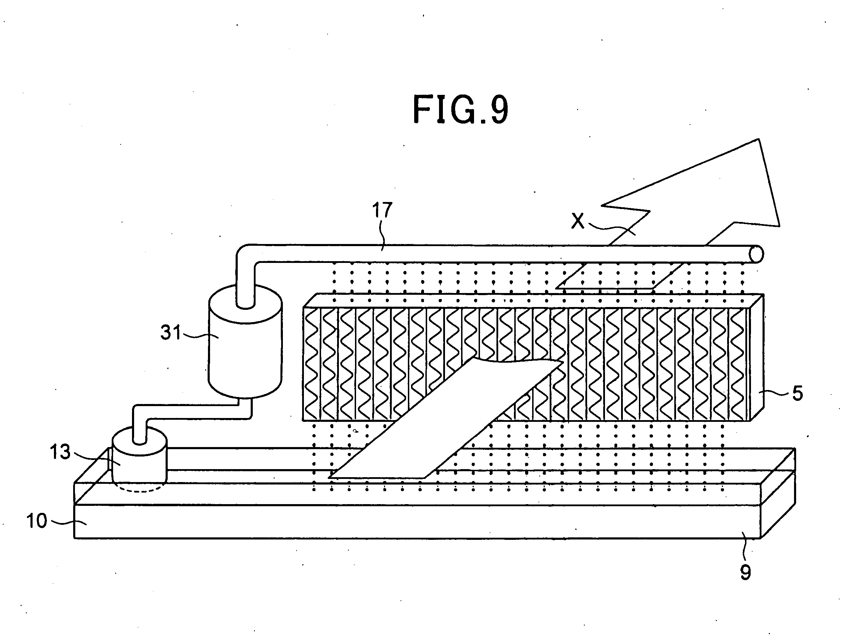

Fig. 9 is a diagram showing air cleaning;
Fig. 10 is a perspective view showing a support tray used in the floor-mount type air filtering apparatus of the embodiment;
Fig. 11 is a perspective view showing a lower portion of a water supply chamber of the floor-mount type air filtering apparatus according to the embodiment;
Fig. 12 is a perspective view showing a state where the support tray used in the floor-mount type air filtering apparatus of the embodiment is secured to the lower portion of the water supply chamber;
Fig. 13 is a partially enlarged view showing an opening portion 8a when the floor-mount type air filtering apparatus shown in Fig. 2 is viewed from the back side;
Fig. 14 is a perspective view showing the internal construction of the floor-mount type air filtering apparatus when a heat sink is provided;
Fig. 15 is a perspective view showing the arrangement of the heat sink; and
Fig. 16 is a diagram showing the arrangement of the heat sink.

### DETAILED DESCRIPTION FO THE PREFERRED EMBODIMENTS

Preferred embodiments according to the present invention will be described hereunder with reference to the accompanying drawings.

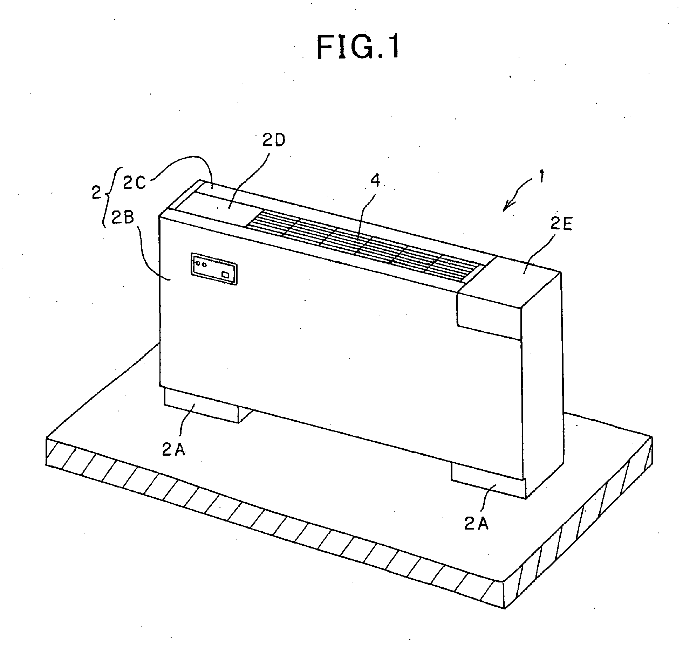

In Fig. 1, reference numeral 1 represents an air filtering apparatus. The following description will be described by representatively using a floor-mount type air filtering apparatus which is used under the state that it is put on the floor, however, the air filtering apparatus is not limited to this type of air filtering apparatus.

The floor-mount type air filtering apparatus 1 has a box-shaped housing 2, and the housing 2 comprises leg pieces 2A, a front panel 2B and a top panel 2C, and an operating lid 2D, an opening/closing lid 2E are arranged in a lateral direction at both the sides of the top panel 2C.

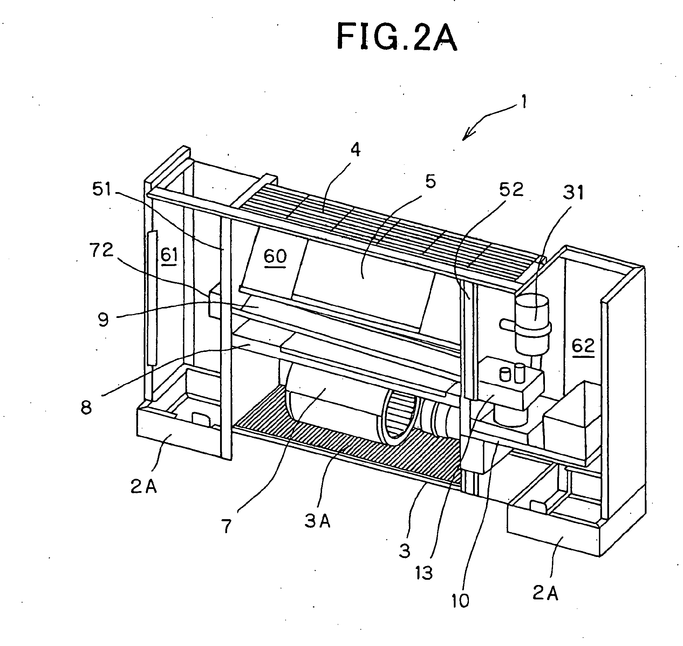

As shown in Fig. 2A, two partition plates 51, 52 extending in the vertical direction are provided, and the inside of the housing 2 is divided into three chambers (an air filtering chamber 60, an electric component chamber 61 and a water supply chamber 62) by the partition plates 51, 52. In this case, the number of the partition plates is not limited to two, that is, the divided chambers is not necessarily limited to three. At least one partition plate may be provided to divide the inside of the housing into at least two chambers.

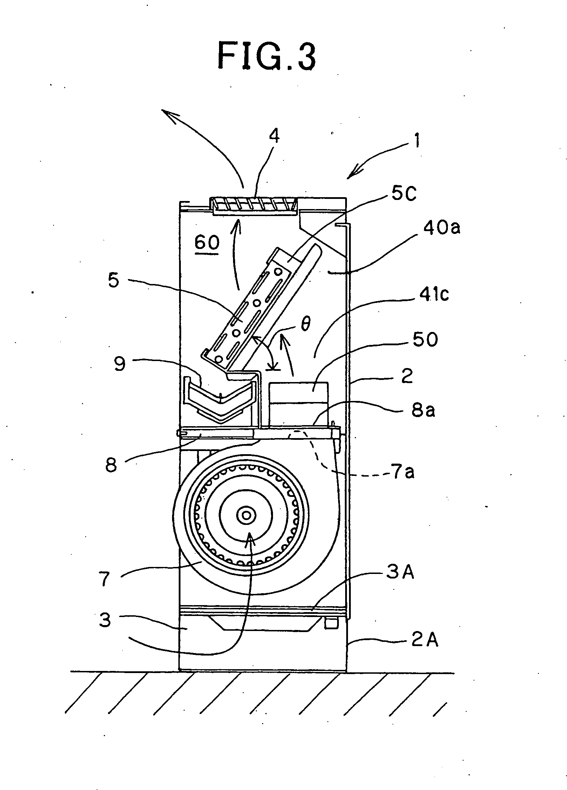

An elongated suction port 3 is formed at the lower side of the air filtering chamber (one chamber) 60 formed at the center of the housing 2, and a pre-filter 3A is disposed at the upper side of the suction port 3. An air blowing fan 7 is disposed above the pre-filter 3A, and a gas-liquid contact member 5 having high water retentivity is disposed above the air blowing fan 7 so as to be tilted as shown in Fig. 3. Furthermore, a water receiving tray 9 for receiving electrolytic water flowing out from the gas-liquid contact member 5 is disposed below the gas-liquid contact member 5. The water receiving tray 9 is designed to have a substantially V-shaped section as shown in Fig. 3.

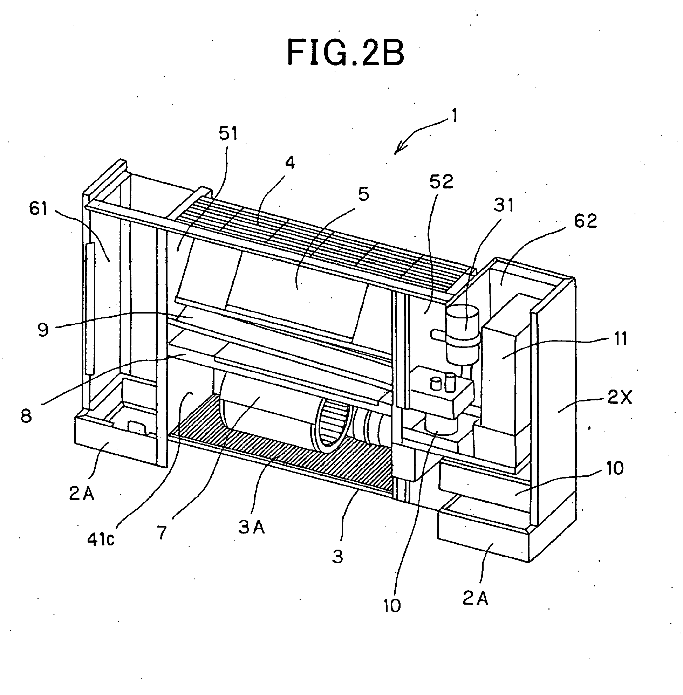

Furthermore, as shown in Figs. 2A and 2B, an elongated blow-out port 4 from which blown air passing through the gas-liquid contact member 5 is blown out is formed above the gas-liquid contact member 5. Reference numeral 8 represents a support plate of the air blowing fan 7, and this support plate 8 is suspended substantially horizontally between the partition plates 51 and 52.

The air blowing fan 7 is secured to the support plate 8, and an opening portion 8a penetrating through the support plate 8 in the vertical direction is formed at the center position (see Fig. 3) in the right and left direction of the gas-liquid contact member 5 on the plane of the support plate 8. The air blow-out port 7a of the air blowing fan 7 is disposed so as to face the opening portion 8a, and air blown out from the air blowing fan 7 is upwardly blown out. The opening portion 8a and the air blow-out port 7a of the air blowing fan 7 are designed to be substantially equal to each other in shape and size. Furthermore, the aperture area of each of the air blow-out port 7a of the air blowing fan and the opening portion 8a is set to be smaller than the horizontal project area of the gas-liquid contact member 5 located above the opening portion 8a.

Electrical parts such as a print board (control board) for controlling the operation of the floor-mount type air filtering apparatus 1, etc. are accommodated in the electric component chamber (the other chamber) 61 formed at the left side of the housing 2. In addition, an electrolytic water supply unit comprising a water supply tank support tray (support tray) 10 for stocking electrolytic water received by the water receiving tray 9, a circulating pump 13 for pumping up the electrolytic water stocked in the support tray 10, an electrolytic bath in which the water pumped by the circulating pump 13 is electrolyzed to generate electrolytic water, a detachable water supply tank 11, etc. is disposed in the water supply chamber 62 formed at the right side of the housing 2 as shown in Figs. 2A and 2B. Fig. 2A shows a state where the water supply tank 11 and a part of the water supply tank support tray 10 shown in Fig. 2B are detached.

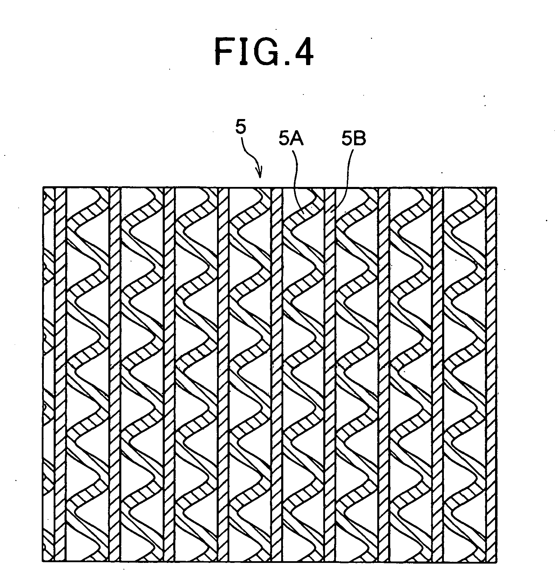

The gas-liquid contact member 5 comprises a filter member having a honeycomb structure, and it is designed so that a broad gas-liquid contact area can be secured, electrolytic water can be dropped and clogging occurs hardly. That is, as shown in Fig. 4, the gas-liquid contact member 5 comprises corrugated raw materials 5A and flat-plate type raw materials 5B joined to the corrugated raw materials 5A, and it is designed in a honeycomb structure as a whole. These raw materials 5A and 5B are formed of a material having little reactivity to electrolytic water described later, that is, a material which is hardly deteriorated by electrolytic water, such as a polyolefin resin group, a PET resin group, a vinyl chloride resin group, a fluorocarbon polymer group, a ceramic resin group or the like. The inclination (tilt) angle θ of the gas-liquid contact member 5 is preferably set to 30° or more. If the inclination angle θ is less than 30°, dropped electrolytic water does not flow along the slope of the gas-liquid contact member 5, but falls downwardly. On the other hand, if the inclination angle θ approaches to 90°, the air flow passage of the air passing through the gas-liquid contact member 5 is nearly horizontal, and thus it is more difficult to blow out the air upwardly. When the air blow-out direction concerned is set to the horizontal direction, it is impossible to blow out air to a remote place, and thus the apparatus is unsuitable for air filtering in a large space. Accordingly, it is preferable that the inclination angle θ satisfies 80°>θ>30°, and in this embodiment, the inclination angle θ is set to about 40°.

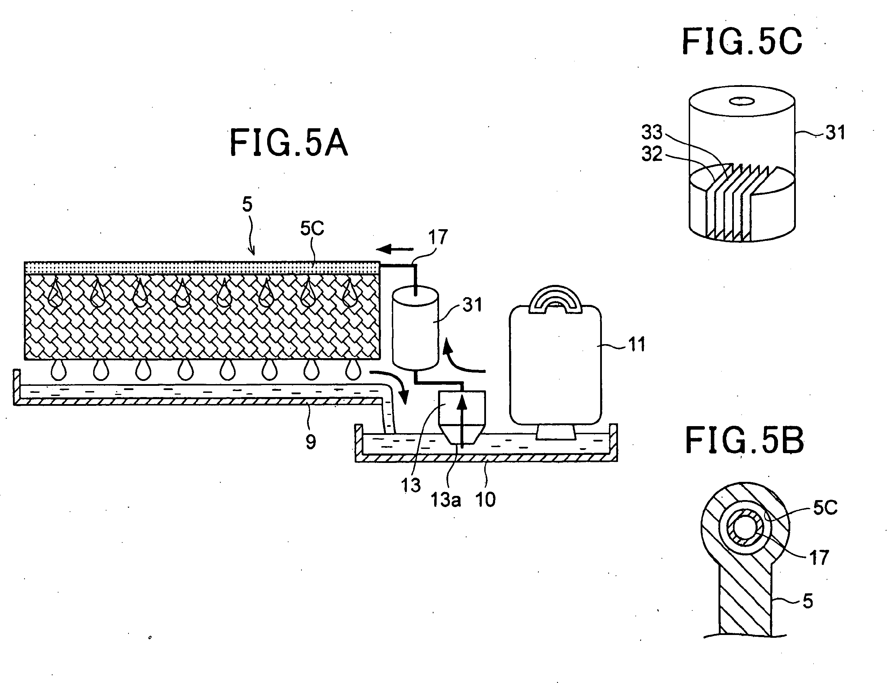

Figs. 5A to 5C show a mechanism for dropping electrolytic water to the gas-liquid contact member 5.

The water receiving tray 9 is disposed below the gas-liquid contact member 5 as described above, and the water supply tank support tray (hereinafter referred to as "support tray") 10 intercommunicates with the water receiving tray 9 or the support tray 10 is disposed at the lower side of the water receiving tray 9.

In this construction, the water receiving tray 9 is disposed so as to be downwardly inclined to the support tray 10, and thus electrolytic water received by the water receiving tray 9 more easily flows to the support tray 10. In this case, the support tray 10 is disposed at the downstream side of the water receiving tray 9.

In this case, the water supply tank 11 for supplying (supplementing) water containing chlorine ions (for example, tap water) and the circulating pump 13 are disposed in the support tray 10. The electrolytic bath 31 of the electrolytic water generating unit is connected to the circulating pump 13, and an electrolytic water supply pipe 17 is connected to the electrolytic bath 31. The electrolytic water supply pipe 17 has many water spray holes (not shown) on the outer peripheral portion thereof, and it is inserted in a water spray portion (box) 5C formed at the upper edge portion of the gas-liquid contact member 5 as shown in Fig. 5B.

As shown in Fig. 5C, the electrolytic bath 31 has plural pairs of electrodes 32 and 33. By supplying current to these electrodes 32 and 33, water flowing into the electrolytic bath 31 is electrolyzed to generate active oxygen species. Here, the active oxygen species are defined as molecules having higher oxidizing activity than normal oxygen molecules and relating materials, and contain so-called narrowly-defined active oxygen such as superoxide anion, singlet oxygen, hydroxyl radical, hydrogen peroxide, etc. and also so-called broadly-defined active oxygen such as ozone, hypohalous acid, hypochlorous acid, etc. The electrolytic bath 31 is disposed in proximity to the gas-liquid contact member 5, and it immediately supply the gas-liquid contact member 5 with active oxygen species generated by electrolyzing water pumped by the circulating pump 13.

Each of the electrodes 32 and 33 is an electrode plate comprising a base of Ti (titan) and a coating layer of Ir (iridium), Pt (platinum), and the current value applied to the electrodes 32, 33 is set to several mA to several tens mA (milliampere) /cm² (square centimeter) in current density to generate a predetermined free residual chlorine concentration (for example, 1mg (milligram)/l (liter)).

By supplying current to tap water through the electrodes 32, 33, at the cathode, the following reaction occurs:

4H⁺ + 4e⁻ + (40H⁻) → 2H₂ + (40H⁻)

At the anode, the following reaction occurs:

2H₂O → 4H⁺ + O₂ + 4e⁻

At the same time, chlorine ions contained in water (pre-added in tap water) make the following reaction:

2Cl⁻ → Cl₂ + 2e⁻

Furthermore, Cl₂ reacts with water as follows:

Cl₂ + H₂O → HClO + HCl

In this construction, HClO (hypochlorous acid) having high sterilization power is generated by supplying current to the electrodes 32, 33, and various microorganisms such as bacteria, virus, etc. can be prevented from breeding in the gas-liquid contact member 5 by making air pass through the gas-liquid contact member 5 to which electrolytic water containing hypochlorous acid is supplied. Therefore, microorganisms such as virus, bacteria, etc. floating in the air passing through the gas-liquid contact member 5 can be inactivated. Odor also reacts with hypochlorous acid in the electrolytic water when passing through the gas-liquid contact member 5, so that it is ionized and dissolved in the electrolytic water. Therefore, the odor can be removed from the air and thus the air is deodorized.

In order to effectively inactivate microorganisms such as virus, bacteria, etc. floating in the air, it is preferable that the contact area between air and electrolytic water in the gas-liquid contact member 5 is increased. Therefore, according to this embodiment, from the viewpoint of increasing the contact area, it is preferable that the gas-liquid contact member 5 is disposed so as to extend substantially from end to end in the lateral direction in the compartmented chamber (i.e., the air filtering chamber 60).

Therefore, the water receiving tray 9 is required to be water-tightly fixed to the partition plate 51 so that no electrolytic water leaks from the gap between the partition plate 51 and the water receiving tray 9. Here, it may be considered that the water receiving tray 9 and the partition plate 51 are welded to each other to water-tightly fix the water receiving tray 9 to the partition plate 51. However, in the case of the welding work, the welding portion is easily oxidized by electrolytic water and also the welding work is cumbersome.

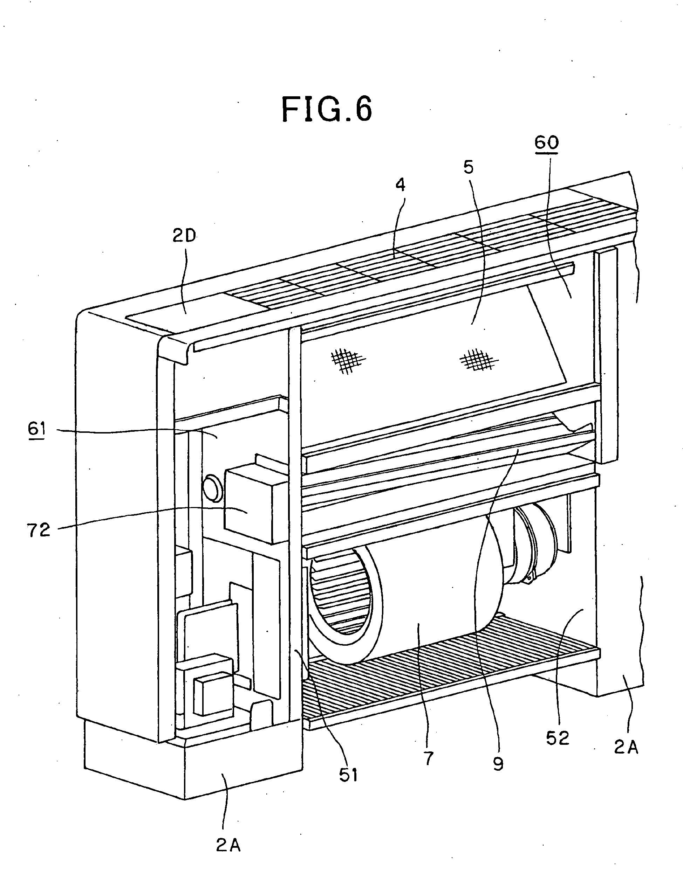

In order to facilitate the fixing work between the water receiving tray 9 and the partition plate 51, it may be also considered that the water receiving tray 9 disposed below the gas-liquid contact member 5 is disposed so that one end thereof at the electric component chamber (61) side is higher than the other end thereof at the water supply chamber (62) side as shown in Fig. 6 (that is, the water receiving tray 9 is obliquely downwardly disposed from the electric component chamber side to the water supply chamber side), and also the upper end portion (end portion) 9A of the water receiving tray penetrates through the partition plate 51 so as to extend to the electric component chamber (61) side so that no electrolytic water leaks downwardly from the gap between the water receiving tray 9 and the partition plate 51.

The above constriction will be described hereunder in more detail with reference to Figs. 6 to 8.

A sponge type seal member 74 is attached to the back side of the water receiving tray 9, and an opening 51A having substantially the same shape as the cross-sectional shape of the water receiving tray 9 (containing the seal member 74) (in this embodiment, substantially V-shape) is formed in the partition plate 51. The upper end portion 9A of the water receiving tray 9 is disposed so as to penetrate through the opening 51A of the partition plate 51 and extend to the electrical component chamber (61) side.

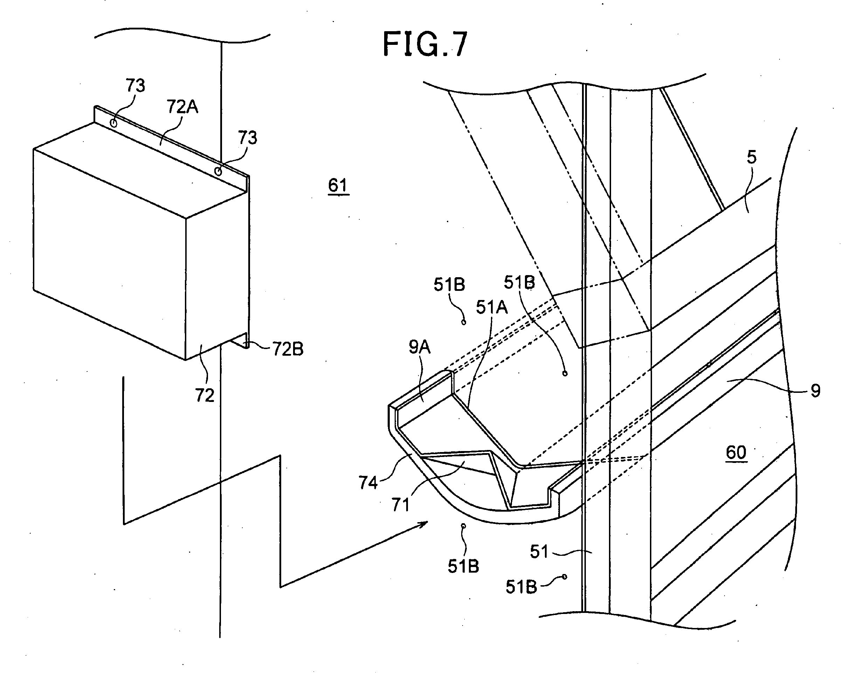

As shown in Fig. 7, a water return portion (throating unit) 71 is formed at the upper end portion 9A of the water receiving tray 9. The water return portion 71 is formed so as to be bent by folding back the lower edge portion of the upper end portion 9A of the water receiving tray 9 upwardly, and it prevents electrolytic water stocked in the water receiving tray 9 from flowing into the electric component chamber 61.

According to the above construction, the water receiving tray 9 is disposed so as to be downwardly inclined from the electric component chamber 61 to the air filtering chamber 60, and thus the electrolytic water in the water receiving tray 9 is prevented from flowing back. Even when electrolytic water is trapped in the water receiving tray 9, the electrolytic water can be prevented from invading into the electric component chamber 61 by the water return portion 7 formed at the upper end portion 9A of the water receiving tray 9.

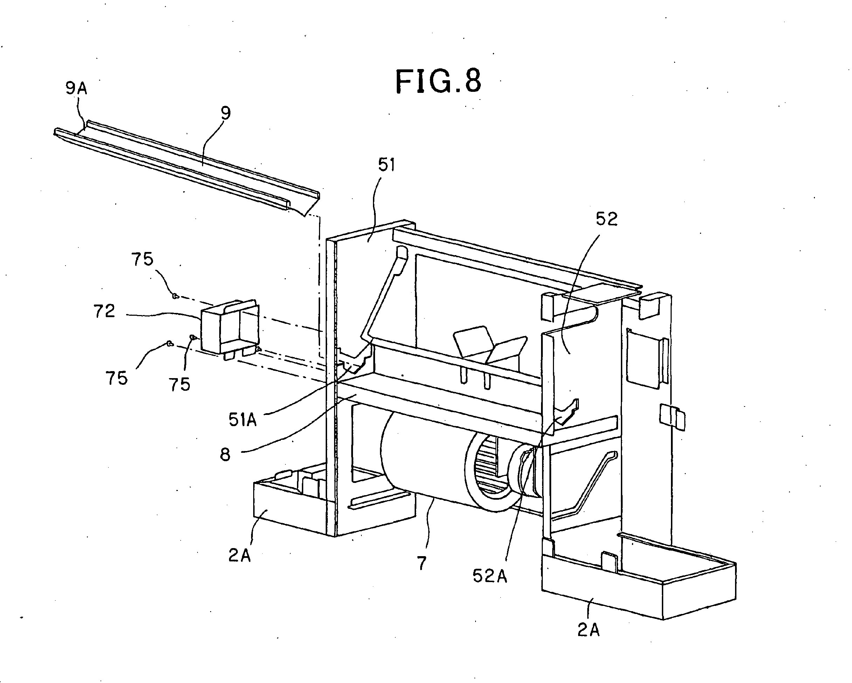

Furthermore, according to the above construction, when the floor-mount type air filtering apparatus is fabricated, the water receiving tray 9 is inserted from the electric component chamber 61 through the opening 51A of the partition plate 51 to the air filtering chamber 60, and then the other end portion of the water receiving tray 9 is inserted through an opening 52A formed in the partition plate 52 so as to extend to the water supply chamber 62 side as shown in Fig. 8, whereby the water receiving tray 9 is secured to the partition plates 51 and 52.

Furthermore, as shown in Figs. 6 to 8, a cover member 72 covering the upper end portion 9A of the water receiving tray 9 may be fixed at one side surface of the partition plate 51 which faces the electrical component chamber (61) side. This cover member 72 is provided with fixing pieces 72A and 72B extending upwardly and downwardly from both the upper and lower surfaces of the cover member 72 respectively, and fixing holes 73 are formed in these fixing pieces 72A and 72B. Furthermore, screw holes 51B are formed at the positions corresponding to the fixing holes 73 in the partition plate 51, and the cover member 72 is fixed by screws 75 (see Fig. 8). The cover member 72 prevents air containing electrolytic water from invading from the air filtering chamber 60 through the opening 51A into the electrical component chamber 61.

Furthermore, the surfaces of the water receiving tray 9, the partition plate 51 and the partition plate 52 are subjected to antirust coating such as cation coating or the like. In general, hypochlorous acid has a property of oxidizing and rusting metal such as iron, etc. Therefore, according to the above construction, the antirust coating is applied to the surface of the water receiving tray 9 receiving electrolytic water at all times and the surfaces of the partition plates 51 and 52 adjacent to the water receiving tray 9, thereby preventing occurrence of rust in the water receiving tray 9 and the partition plates 51 and 52.

Still furthermore, according to this embodiment, as described above, the water receiving tray 9 is secured to the partition plates 51 and 52 by inserting the water receiving tray 9 into the openings 51A and 52A. According to this construction, the effect of the antirust coating is prevented from being lost by heat of welding, and also occurrence of rust in the water receiving tray 9 and the partition plates 51 and 52 can be prevented. Furthermore, antirust coating is applied to the water receiving tray 9 and the partition plates 51 and 52 in advance, so that it is unnecessary to newly apply antirust processing to the joint portions of these parts, and the assembly work of these members can be easily performed. In this embodiment, the water receiving tray 9 and the partition plates 51 and 52 are subjected to antirust coating. However, the water receiving tray and the partition plates may be formed of stainless steel (sus304).

Next, the operation of the floor-mount type air filtering apparatus of this embodiment will be described.

In Fig. 1, when the operating lid 2D is opened, an operation panel (not shown) is exposed. By operating this operation panel, the operation of the floor-mount type air filtering apparatus 1 is started. When this operation is started, the circulating pump 13 is driven, and water (for example, tap water) stocked in the support tray 10 is supplied to the electrolytic bath 31.

In this electrolytic bath 31, the water is electrolyzed by supplying current to the electrodes 32 and 33 to generate electrolytic water containing active oxygen species. This electrolytic water is passed through the spray holes (not shown) of the electrolytic water supply pipe 17, and sprayed into the water spray portion (box) 5C. The electrolytic water thus sprayed infiltrates into the upper edge portion of the gas-liquid contact member 5 and gradually diffuse to the lower portion of the gas-liquid contact member 5.

Surplus electrolytic water is collected in the water receiving tray 9, flows to the adjacent support tray 10 and then is stocked there. In this construction, the water supply system is a circulating system, and when the amount of water is reduced due to vaporization or the like, a suitable amount of water (tap water or the like) is supplemented through the water supply tank 11 into the support tray 10. When the amount of water in the water supply tank 11 is reduced, the opening/closing lid 2E (see Fig. 1) is opened, and the water supply tank 11 is taken out to supplement water (tap water or the like) into the water supply tank 11.

Indoor air is supplied through the air blowing fan 7 to the gas-liquid contact member 5 filled with the electrolytic water as indicated by an arrow X (see Fig. 3). The indoor air is brought into contact with the active oxygen species of the electrolytic water infiltrating in the gas-liquid contact member 5 and then blown out to the room again. For example, when influenza virus inducing infection is floated in the indoor air, the active oxygen species has a function of breaking and vanishing (removing) the surface proteins of the virus. When influenza virus is broken, the influenza virus is not bonded to a receptor which is required for the influenza virus to infect, and thus the infection can be prevented. As a demonstration test result, it has been found that when air having influenza virus floated therein is passed through the gas-liquid contact member 5 of this embodiment, 99% or more of influenza virus can be removed (inactivated).

In this embodiment, the inside of the floor-mount type housing 2 is divided into the air filtering chamber 60 and the electric component chamber 61 (and/or the water supply unit 62) by the partition plate 51 (and/or the partition plate 52), and the gas-liquid contact member 5 which is disposed substantially from end to end in the lateral direction and supplied with electrolytic water from the electrolytic bath 31, and the air blowing fan 7 for blowing indoor air to the gas-liquid contact member 5 are provided in the air filtering chamber 60. Furthermore, the controller for controlling the air blowing fan 7, the electrolytic bath 31, etc. is provided in the electronic component chamber 61. Furthermore, the water receiving tray 9 for receiving electrolytic water flowing out from the gas-liquid contact member 5 is disposed below the gas-liquid contact member 5, and the end portion 9A of the water receiving tray 9 penetrates through the partition plate 51 and extends to the electric component chamber 61. Therefore, no gap occurs between the partition plate 51 and the water receiving tray 9, and the water receiving tray 9 can be water-tightly secured to the partition plate 51. Furthermore, according to this embodiment, the water receiving tray 9 is inserted into the opening 51A formed in the partition plate 51 and secured to the partition plate 51, and thus it is unnecessary to carry out a welding work or screw clamping work, and thus the water receiving tray 9 can be simply secured to the partition plate 51.

In this embodiment, the cover member 72 covering the end portion 9A of the water receiving tray 9 is secured to the electric component chamber (61) side of the partition plate 51, and thus air containing electrolytic water is prevented from flowing from the air filtering chamber 60 through the opening 51A of the partition plate 51 into the electric component chamber 61. Therefore occurrence of rust in the electrical parts in the electrical component chamber 61 can be prevented. Furthermore, according to this embodiment, the surface of the water receiving tray 9 and the surface of the partition plate 51 are subjected to antirust coating such as cation coating or the like, and thus occurrence of rust in the water receiving tray 9 receiving electrolytic water at all times and the partition plate 51 adjacent to the water receiving tray 9 can be prevented.

Furthermore, according to this embodiment, indoor air sucked from the suction port 3 provided at the lower portion of the housing 2 is brought into contact with electrolytic water dropped to the gas-liquid contact member 5, and then blown out from the air blow-out port 4 provided at the upper portion of the housing 2. Therefore, even when the floor-mount type air filtering apparatus 1 is set up in a so-called large space such as a kindergarten, an elementary/junior/high school, long-term care insurance facilities, a hospital or the like, indoor air which has been brought into contact with electrolytic water and thus subjected to sterile filtration can be blown out to a remote place in a large space, so that the air filtering operation in a large space can be effectively performed.

Still furthermore, according to this embodiment, electrolytic water containing hypochlorous acid, etc. is collected in the water receiving tray 9, and flows to the adjacent support tray 10. Therefore, no microorganism occurs in each tray and thus occurrence of slime is prevented. Accordingly, the cleaning and maintenance frequency of each tray can be reduced, and the labor of maintenance, etc. can be reduced.

In the above construction, it is desirable to adjust the concentration of active oxygen species (hypochlorous acid) in electrolytic water to a target concentration. The target concentration is normally set to a value enough to inactivate microorganisms such as virus, bacteria, fungus, etc. which frequently exist in a set-up place of the floor-mount type air filtering apparatus (for example, school). In this case, the concentration of the active oxygen specifies (for example, hypochlorous acid) in the electrolytic water can he adjusted by changing the current flowing through the electrodes 32 and 33 or the voltage applied between the electrodes 32 and 33. For example, by increasing the current flowing through the electrodes 32 and 33 (for example, 40mA/cm² in current density), the concentration of hypochlorous acid can be changed to a high value. Accordingly, the concentration of active oxygen species can be changed by merely adjusting the voltage applied to the electrodes or adjusting the current flowing through the electrodes without using any new device, and thus the cost can be reduced and the space can be saved.

Here, condensed materials filtered from air by electrolytic water is precipitated at the bottom portion of the support tray, and thus it is preferable to periodically detach the support tray from the air filtering apparatus and clean the support tray. However, the circulating pump 13 is inserted in the support tray as shown in Fig. 5A, and thus when the support tray is drawn out from the inside of the housing to the front side of the air filtering apparatus, the support tray and the circulating pump interfere with each other. Therefore, the support tray is not smoothly detached from the housing. In order to smoothly detach the support tray 10 from the air filtering apparatus, the following construction may be adopted.

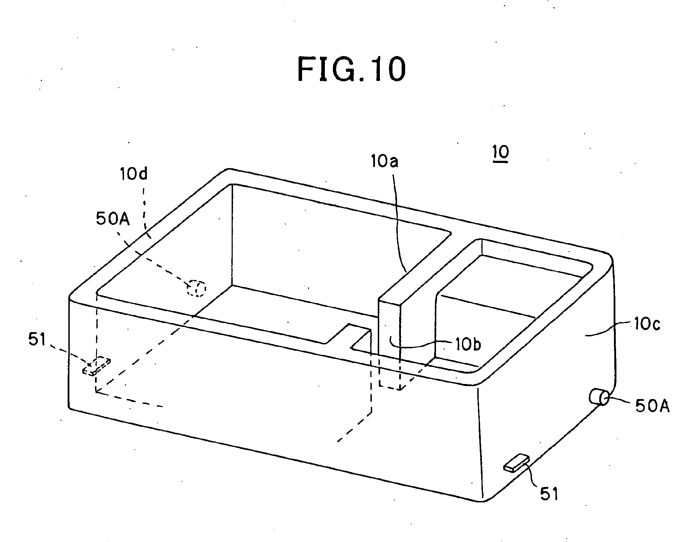

Fig. 10 is a perspective view showing a support tray 10.

The support tray 10 is formed of foamed material, and designed to have a substantially rectangular parallelepiped outer shape. The support tray 10 is designed to be hollow, and has an opened upper surface. Furthermore, the inside of the support tray 10 is divided into right and left spaces by a partition wall 10a. The circulating pump 13 is inserted in the support tray 10 so that the water suction port 13a of the circulating pump 13 is located in the left space, and also the water supply tank 11 is supported in the right space. The partition wall 10a has a groove 10b formed at the center portion thereof, and electrolytic water freely flows in the right-and-left direction.

A projecting portion 50A is provided at the lower portion of the back side of the right surface portion 10c of the support tray 10, and also a support tag 51 is provided at the lower portion of the front side of the right side surface portion 10c so that the flat portions thereof face in the vertical direction. The projecting portion 50A and the support tag 51 project outwardly from the right surface portion 10c, and they are formed integrally with the support tray 10 in the plastic foam molding process. Furthermore, the projecting portion 50A and the support tag 51 may be also provided to the left surface portion 10d of the support tray 10 so as to be symmetric with those of the right surface portion 10c.

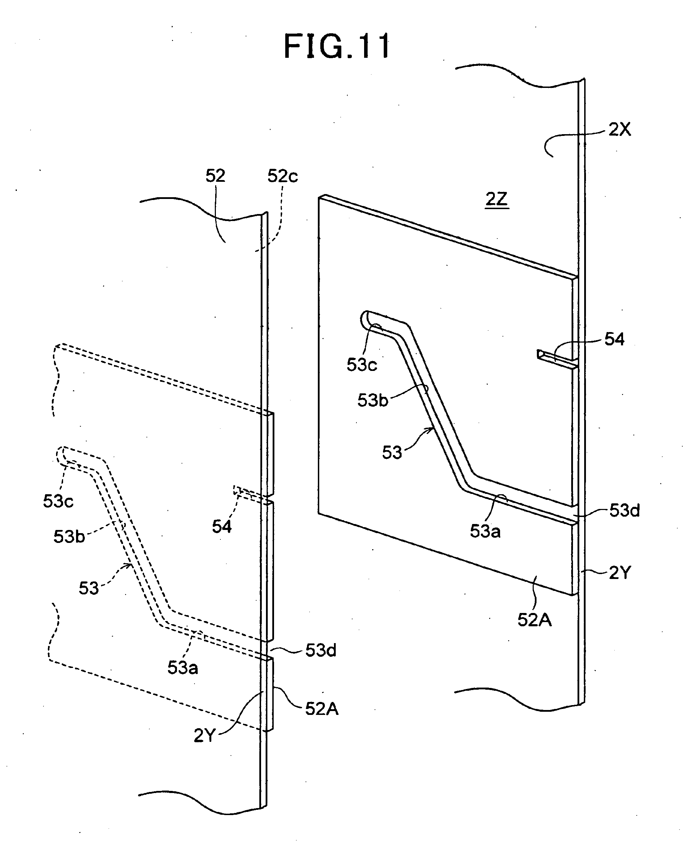

Fig. 11 is a perspective view showing a lower portion of the water supply chamber 62 to which the support tray 10 is secured. In the water supply chamber 62, the left surface (wall) 52c of the water supply chamber 62 is formed by the partition plate 62, and the right side surface (wall) 2Z of the water supply chamber 62 is formed by the side plate 2X of the housing 2. A pair of guide plates 52A for guiding the support tray 10 when the support tray 10 is attached to or detached from the housing 2 are provided on both the wall surfaces 52c and 2Z. With respect to the guide plates 52, only one guide plate 52 may be provided to one of the wall surfaces 52c and 2Z. A rail groove 53 to which the projecting portion 50A of the support tray 10 is fitted and a holding groove 54 to which the support tag 51 of the support tray 10 is fitted are formed in the guide plate 52.

The rail groove 53 comprises a horizontal movement portion 53a extending horizontally from the surface portion 2Y of the housing 2 in the backward direction of the housing, a vertical movement portion 53b extending upwardly or obliquely upwardly from the back-side end portion of the horizontal movement portion 53a to the back side of the housing, and a fixing groove portion 53c which slightly extends horizontally from the upper end portion of the vertical movement portion 53b in the backward direction of the housing.

The holding groove 54 is located substantially at the same height as the fixing groove portion 53c, and extends horizontally from the surface portion 2Y of the housing 2 in the backward direction.

Next, the operation of the floor-mount type air filtering apparatus having the support tray 10 shown in Fig. 10 will be described.

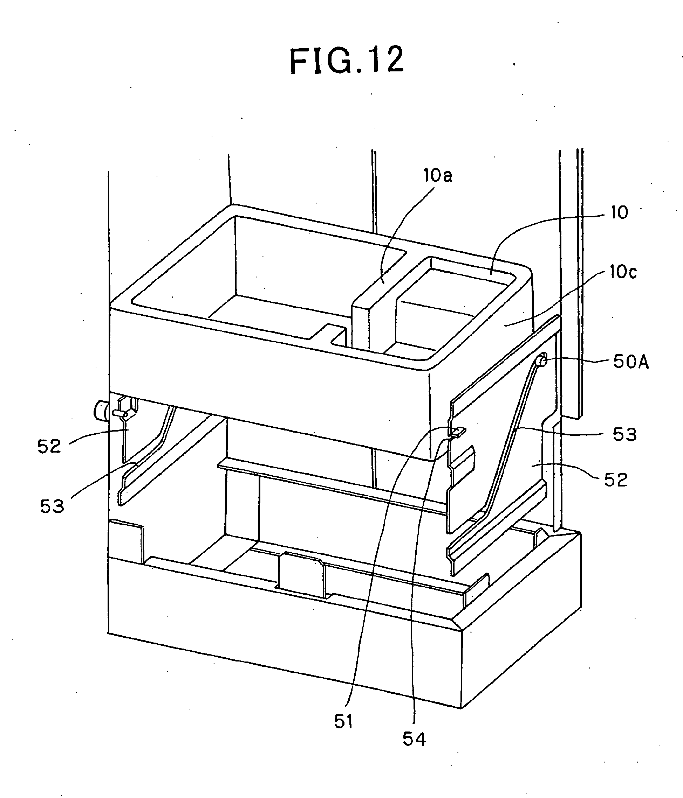

Under the state that the support tray 10 is mounted in the housing 2, the circulating pump 13 and the water supply tank 11 are inserted in the support tray 10 as shown in Fig. 5A. Furthermore, as shown in Fig. 12, when the support tray 10 is mounted in the water supply chamber 62 of the housing 2, the projecting portion 50A is fitted in the fixing groove portion 53c of the rail groove 53, and the support tag 51 is fitted in the holding groove 54.

When the support tray 10 is detached from the above state, the support tray 10 is first horizontally drawn out to the surface portion 2Y side of the housing 2 to release the engagement between the support tag 51 and the holding groove 54. This horizontal movement distance is set so that the inner wall of the support tray 10 and each of the circulating pump 13 and the water supply tank 11 does not interfere with each other.

After the engagement between the support tag 51 and the holding groove 54 is released, the support tray 10 is obliquely downwardly moved while keeping the support tray 10 in a horizontal position. At this time, the projecting portion of the support tray 10 is guided along the extending direction of the vertical movement portion 53b, whereby the support tray 10 is obliquely downwardly moved along a fixed passage. The shape of the support tray 10 and the moving passage of the support tray 10 are determined so that the inner wall of the back side of the support tray 10 and each of the circulating pump 13 and the water supply tank 11 do not interfere with each other. The support tray 10 is moved until the circulating pump 13 and the water supply tank 11 are located at the outside of the support tray 10 under the state that the support tray 10 is guided to the lower end of the vertical movement portion 53b.

Finally, the support tray 10 is horizontally drawn out to the surface portion 2Y side of the housing 2 while keeping the support tray 10 horizontally. At this time, the projecting portion 50A of the support tray 10 is guided along the extending direction of the horizontal movement portion 53a, whereby the support tray 10 is horizontally moved. Then, the projecting portion 50A is detached from an open portion of the rail groove 53, thereby detaching the support tray 10 from the housing 2.

When the support tray 10 is mounted in the housing 2, the support tray 10 is moved in the reverse way to mount the support tray 10 in the housing so that the support tray 10 and each of the circulating pump 13 and the water supply tank 11 do not interfere with each other.

According to the floor-mount type air filtering apparatus, the rail groove 53 is designed so that the projecting portion 50 is guided downwardly along the rail groove 53 until the circulating pump 13 is located at the outside of the support tray 10 when the support tray 10 is detached from the inside of the housing 2. Accordingly, by moving the projecting portion 50 of the support tray 10 along the rail groove 53, the support tray can be detached while the projecting portion 50 of the support tray 10 and the circulating pump 13 do not interfere with each other.

Furthermore, the support tray 10 is obliquely downwardly guided along the vertical movement portion 53b from the state that the support tray 10 is mounted in the housing Therefore, by moving the projecting portion 50 of the support tray 10 along the rail groove 53, the support tray 10 can be detached so that the support tray 10 and the circulating pump 13 do not interfere with each other. Furthermore, the support tray 10 can be drawn out to the take-out side along the horizontal movement portion 53b, and thus the support tray 10 can be horizontally drawn out without the support tray 10 being inclined.

In the above construction, the projecting portion 50 is provided to the support tray 10, and the rail groove 53 is provided to the housing 2 (partition plate 62) side. However, the rail groove 53 may be provided to the support tray 10 while the projecting portion 50 is provided to the housing 2 (partition plate 62) side. Accordingly, the support tray 10 can be detached while the support tray 10 and the circulating pup 13 do not interfere with each other.

Furthermore, the vertical movement portion 53b is formed so as to be inclined, however, it may be formed so as to be vertical. Accordingly, the support tray 10 can be detached so that the support tray 10 and the circulating pup 13 do not interfere with each other.

Still furthermore, it is preferable in the floor-mount type air filtering apparatus that indoor air blown out from the air blowing fan is evenly applied to the whole of the gas-liquid contact member. In order to satisfy this condition, the air blowing fan may be designed to have an air blowing port having the same outer shape as the gas-liquid contact member. However, the air blowing fan whose air blowing port has a large aperture area as described above needs a large set-up space, and thus it makes it difficult to miniaturize the air filtering apparatus.

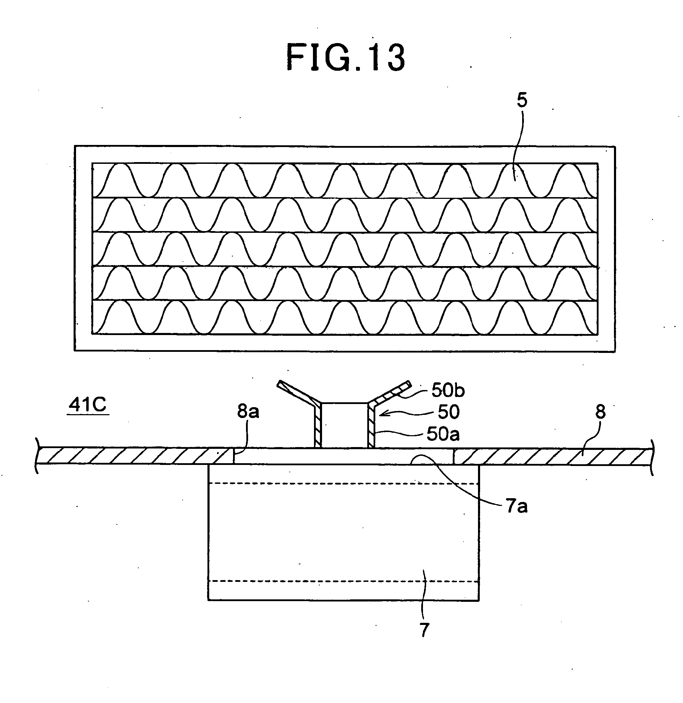

Fig. 13 shows a floor-mount type air filtering apparatus having a mechanism for enabling indoor air blown out from the air blowing fan to be evenly applied to the whole of the gas-liquid contact member all over without increasing the scale of the air filtering apparatus, and corresponds to a back side view of the floor-mount air filtering apparatus of Fig. 2. In Fig. 13, the view of the opening portion 8a is enlarged.

As shown in Figs. 3 and 13, a pair of air guide plates 50 may be provided at the opening portion 8a so as to be symmetrically disposed in the right-and-left direction in Fig. 13. The lower side end portions of the air guide plates 50 are fixed to the support plate 8. Each air guide plate 50 comprises an extension portion 50a extending upwardly from the lower end portion thereof, and a guide portion 50b extending obliquely upwardly from the upper end portion of the extension portion 50a toward the gas-liquid contact member 5 located at a remote place from the air blowing fan 7. The extension portion 50A and the guide portion 50b of the air guide plate 50 are formed integrally with each other by folding sheet metal.

In this case, air blown out upwardly from the air blow-out port 7a of the air blowing fan 7 is further upwardly blown out from the opening portion 8a of the support plate 8, and upwardly guided along the extension portion 50a of the air guide plate 50. At this time, the air blown out along the extension portion 50a is upwardly guided with no occurrence of turbulent flow or eddy and also without reducing the air flow rate and the air flow amount.

The air guided along the extension portion 50a is further guided along the inclination of the guide portion 50b toward the gas-liquid contact member 5 located at a remote place from the air blowing port 7a of the air blowing fan 7.

Here, an experiment result confirming the effect of the air guide plate will be described.

When air was blown from the air blowing fan 7 at an air flow rate of about 3.5m/sec to 3.8m/sec under the state that no air guide plate 50 was provided, the air flow rate at the gas-liquid contact member 5 located at the outside of the air blowing port 7a of the air blowing fan 7 was equal to about 0.3m/s to 0.4m/sec.

Next, when air was blown from the air blowing fan 7 at an air flow rate of about 3.5m/sec to 3.8m/sec under the state that no extension portion 50a was provided to the air guide plate 50, the air flow rate at the gas-liquid contact member 5 located at the outside of the air blowing port 7a of the air blow fan 7 was equal to 1m/sec or less.

On the other hand, when air was blown out from the air blowing fan 7 at an air flow rate of about 3.5m/sec to 3. 8m/sec under the state that the extension portion 50a was provided to the air guide plate 50, the air flow rate at the gas-liquid contact member 5 located at the outside of the air blowing port 7a of the air blowing fan 7 exceeded 1m/sec. Furthermore, the air flow rate at the center portion which was not displaced from the opening portion 7a of the air blowing fan 7 was kept to about 3.5m/sec to 3.8m/sec.

According to the floor-mount type air filtering apparatus of this embodiment, air blown out upwardly from the air blowing port 7a of the air blowing fan 7 is upwardly guided toward the gas-liquid contact member 5 by the air guide plates, whereby neither turbulent flow nor eddy occurs in the air stream and also the air flow rate and the air flow amount of the air blown out from the air blowing fan 7 can be secured. Furthermore, by providing the guide portion 50b, the air can be fed from the air blowing port 7a of the air blowing fan 7 toward the gas-liquid contact member 5 located at a remote place.

In the above-described embodiment, the extension portion 50A and the guide portion 50b of the air guide plate 50 are formed integrally with each other. However, they may be formed separately from each other. In this case, they are detachably assembled with each other by screws or the like. Furthermore, a plurality of guide portions 50b which are different in inclination angle may be prepared and selectively used, so that the inclination angle of the air guide plate 50 being used can be freely adjusted. Accordingly, the flow direction of air to be blown to the gas-liquid contact member 5 can be adjusted.

Furthermore, the position of the guide portion 50b may be designed to be slidable in the vertical direction, so that the vertical length of the extension portion 50a can be increased. Accordingly, the flow direction of air to be blown to the gas-liquid contact member 5 can be also adjusted.

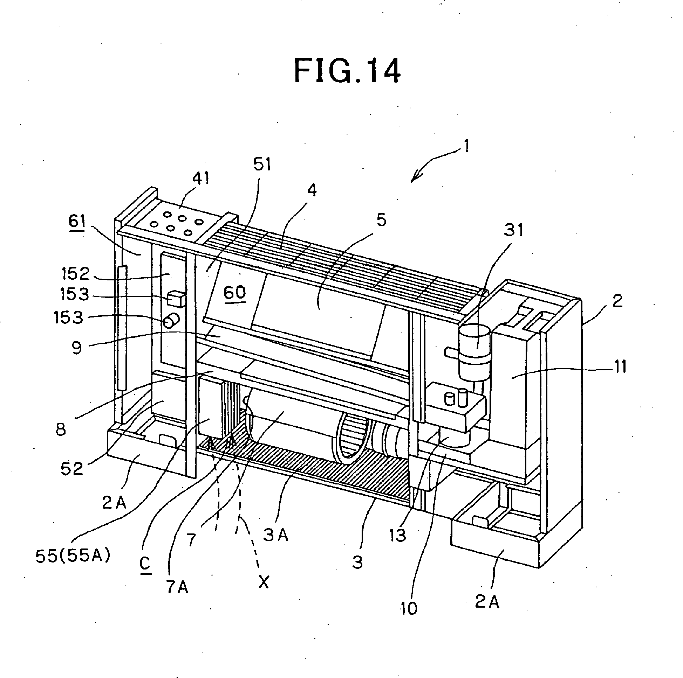

As described above, the electrical component chamber 61 is water-tightly formed by the partition plate (partition member) 51, and a control board for controlling the whole of the apparatus, etc. are accommodated in the electrical component chamber 61. Fig. 14 shows the arrangement of the control board, etc. in the electrical component chamber 61. A control board portion for controlling the electrolytic water generating unit for generating electrolytic water is also arranged in the electrical component chamber 61 (or electrical component box). In this case, electrical parts generating high heating values are mounted on the control board portion for the electrolytic generating unit, and thus it is preferable to efficiently radiate heat. In order to radiate heat from the control board portion as described above, a heat sink may be used as a heat radiating mechanism.

The heat radiating mechanism will be described in detail with respect to Figs. 14 to 16.

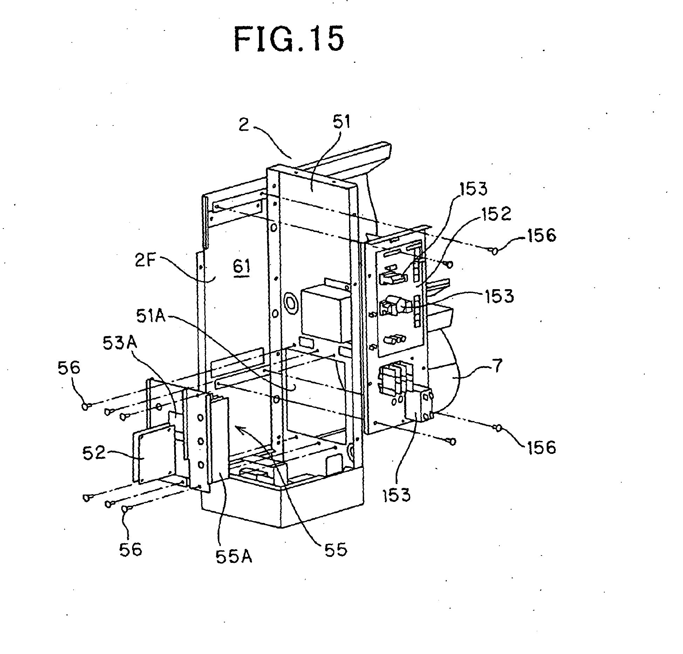

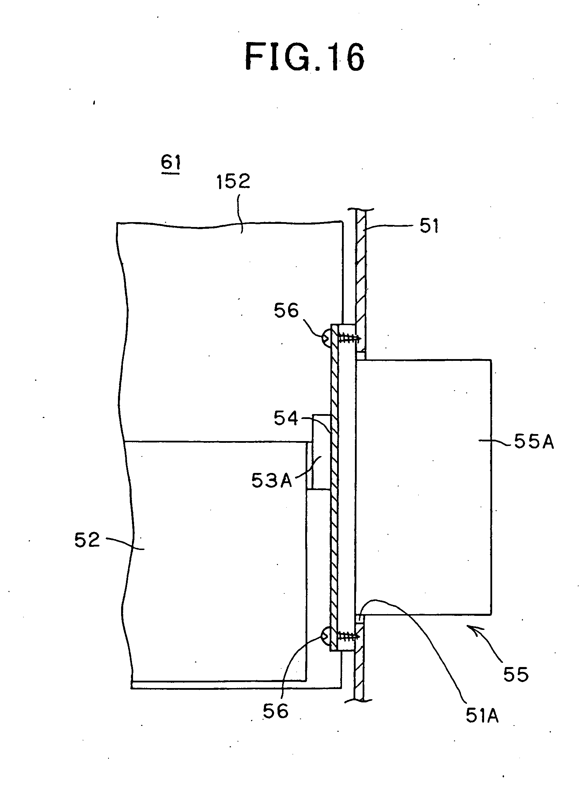

As shown in Fig. 14, control boards 152 and 52 for controlling the whole apparatus are arranged in the electrical component chamber 61. An electrical part 153 is mounted on one control board 152, and the control board 152 is fixed to the back plate 2F of the housing 2 through screws 156 (see Fig. 15). Furthermore, as shown in Fig. 16, a switching transistor (electrical part) 53A for controlling current supply to the electrodes 32 and 33 is connected to the other control board 52, and the transistor 53A is secured through an insulating sheet 54 to a heat sink 55 for heat radiation. The heat sink 55 is formed of aluminum by extrusion molding, and secured to the partition plate 51 through screws 56.

The heat sink 55 has plural fin members 55A, and an opening 51A having substantially the same outer shape as the assembly of the plural fin members 55A is formed in the partitionplate 51 as shown in Fig. 14. The plural finmembers 55A penetrates through the opening 51A and extends to the suction space C between the pre-filter 3A and the support plate 8 at the lower stage of the air filtering chamber 60 so as to face the suction port 7A of the air blowing fan 7 (see Fig. 14).

According to this construction, indoor air flows in the suction space C while coming into contact with the fins 55A of the heat sink 55, and then sucked into the suction port 7A of the air blowing fan 7. This air passes and flows between the respective fin members 55A, whereby the cooling of the heat sink 55 is promoted and the heat radiation of the electrical part 53A is particularly promoted. The electrical part 53A is an electrical part for controlling the current supply to the electrodes 32 and 33, and the heat generation amount thereof is large. However, according to this construction, the heat radiation amount is increased, and thus the electrical part 53A is sufficiently cooled. Furthermore, since the heat sink 55 is disposed so as to penetrate through the opening 51A of the partition plate 51, the heat sink 55 may be inserted from the electric component chamber 61 into the air filtering chamber 60 while the electrical part 53A is mounted on the heat sink 55 in advance, and then secured to the partition plate 51 by screws 56. Therefore, the installation of the heat sink 55 can be enhanced.

The present invention is not limited to the above embodiments, and various modifications may be made without departing from the subject matter of the present invention. For example, ozone (O₃), hydrogen peroxide (H₂O₂) or the like may be generated as active oxygen specifies. In this case, when platinum tantalum electrodes are used as the electrodes 32 and 33, active oxygen species can be highly efficiently and stably generated from water containing diluted ion species by electrolysis.

At this time, at the anode, the following reaction occurs:

2H₂O → 4H⁺ + O₂ + 4e⁻

Simultaneously with the above reaction, the following reactions occur, and ozone (O₃) is generated.

3H₂O → O₃ + 6H⁺ + 6e⁻

2H₂O → O₃ + 4H⁺ + 4e⁻

Furthermore, at the cathode, the following reactions occur:

4H⁺ + 4e⁻ + (40H⁻) → 2H₂ + (40H⁻)

O₂⁻ + e⁻ + 2H⁺ → H₂O₂

That is, O₂⁻ generated through the electrode reaction and H⁺ in solution are bonded to each other to generate hydrogen peroxide (H₂O₂).

In this construction, ozone (O₃) and hydrogen peroxide (H₂O₂) which have strong sterilizing power are generated by supplying current to the electrodes, and electrolytic water containing ozone (O₃) and hydrogen peroxide (H₂O₂) can be created. The concentration of ozone or hydrogen peroxide in the electrolytic water is adjusted to a value suitable for inactivate target virus or the like and air is passed through the gas-liquid contact member 5 supplied with the electrolytic water having this concentration, whereby target virus, etc. floating in the air can be inactivated. Furthermore, even odor reacts with ozone or hydrogen peroxide in the electrolytic water when passing through the gas-liquid contact member 5, and ionized and dissolved in the electrolytic water, whereby the odor components are removed from the air and thus the air is deodorized.

When scale is deposited on the electrode (cathode) due to electrolysis of tap water, it is difficult to continue the electrolysis because the electrical conductivity is lowered.

In this case, it is effective to invert the polarities of the electrodes (switching the plus and minus polarities of the electrodes). The scale deposited on the cathode can be removed by performing electrolysis while the cathode is used as the anode. Under the polarity inverting control, the polarities of the electrodes maybe inverted periodically by using a timer, or the polarities of the electrodes may be inverted irregularly, for example, every time the operation of the apparatus is started or the like. Furthermore, increase of the electrolysis resistance (reduction in electrolysis current or increase in electrolysis voltage) may be detected, and the polarities of the electrodes maybe inverted on the basis of the detection result.

The above embodiment adopts the water supply system based on the water supply tank 11 which can be freely put in and out. However, the present invention may adopt a water pipe water supply system in which a tap water pipe is connected to the apparatus in place of the water supply tank 11. and city water (tap water) is introduced.

Furthermore, in the above embodiment, the water return portion 71 is formed at the end portion 9A of the water receiving tray 9 by bending the end portion of the water receiving tray 9, however, the present invention is not limited to this embodiment. For example, a bank may be provided to the end portion 9A of the water receiving tray 9 to prevent flow-back of electrolytic water.

Still furthermore, the above-described embodiments adopt an indoor air circulating system in which indoor air is sucked from a room, subjected to sterile filtration (air filtering treatment) and then returned to the room. However, air to be subjected to sterile filtration is not limited to indoor air, and outdoor air may be directly subjected to sterile filtration and then supplied to the room. Furthermore, both indoor air and outdoor air may be subj ected to sterile filtration, and then supplied to the room. That is, any air in-take manner may be adopted.

Still furthermore, the above-described embodiments, the air filtering apparatus is a floor-mount type air filtering apparatus that is used under the state that it is put on the floor. However, the air filtering apparatus of the present invention is not limited to the floor-mount type air filtering apparatus, and it may be used in any condition, for example, in a wall-suspended fashion, in a ceiling-suspended fashion, in a ceiling-cassette fashion or the like.

## Claims

1. An air filtering apparatus comprising a housing (2), at least one partition plate (51, 52) for partitioning the inside of the housing into at least two chambers (60, 61, 62), a gas-liquid contact member (5) supplied with electrolytic water, a water receiving tray (9) for receiving the electrolytic water flowing out from the gas-liquid contact member, an air blowing fan (7) for blowing air to the gas-liquid contact member so that the air is brought into contact with the electrolytic water supplied to the gas-liquid contact member, and a controller (52, 53) for controlling the air filtering apparatus, **characterized by** further comprising;
an electrolytic water supply unit (31) for generating electrolytic water and supplying the generated electrolytic water to the gas-liquid contact member;
a circulating pump (13) for feeding the electrolytic water received in the water receiving tray (9) to the electrolytic water supply unit (31) so that the electrolytic water circulates successively through the electrolytic water supply unit (31), the gas-liquid contact member (5), the water receiving tray (9) and the circulating pump (13) in this order, wherein the gas-liquid contact member (5) and the air blowing fan (7) are disposed in one of the chambers, and one of the controller and the electrolytic water supply unit is disposed in the other chamber of the at least two chambers.

2. The air filtering apparatus according to claim 1, wherein one end portion (9A) of the water receiving tray (9) penetrates through one partition plate (51) so as to extend to the other chamber (61).

3. The air filtering apparatus according to claim 2, wherein a cover member (72) for covering the end portion (9A) of the water receiving tray (9) is provided in the other chamber (61).

4. The air filtering apparatus according to claim 2, wherein antirust coating is applied to the water receiving tray (9) .

5. The air filtering apparatus according to claim 2, wherein a water return portion (71) is provided to the end portion of the water receiving tray.

6. The air filtering apparatus according to claim 2, wherein the water receiving tray (9) is disposed so as to downwardly incline from the other chamber (61) to the one chamber (60) .

7. The air filtering apparatus according to claim 1, wherein the electrolytic water contains active oxygen species achieved by electrolyzing water, and the active oxygen species contains at least one of hypochlorous acid, ozone and hydrogen peroxide.

8. The air filtering apparatus according to claim 1, wherein the electrolytic water supply unit contains a support tray (10) for receiving electrolytic water flowing from the water receiving tray (9) located below the gas-liquid contact member (5) and stocking the electrolytic water concerned, and the circulating pump (13) is inserted in the support tray and pumps up the electrolytic water stocked in the support tray, wherein one of a projecting portion (50A) and a rail groove (53) in which the projecting portion (50A) is fitted so as to be movable along the rail groove (53) is provided to at least one of both the side surfaces (10c, 10d) of the support tray (10), the other of the projecting portion and the rail groove is provided to at least one of the inner side surface (2Z) of the housing and the side surface (52c) of the partition plate (52), the inner side surface (2Z) and the side surface (52c) facing the side surfaces (10c, 10d) of the support tray (10), and the rail groove (53) is designed so that the projecting portion (50A) is downwardly guided along the rail groove (53) until the circulating pump (13) is located at the outside of the support tray (10).

9. The air filtering apparatus according to claim 8, wherein the rail groove (53) comprises a vertical movement portion (53b) and a horizontal movement portion (53a) intercommunicating with the vertical movement portion (53b), the support tray (10) being movable in one of a downward direction and an obliquely downward direction and horizontally movable along the horizontal movement portion (53a) and detachable from one end of the horizontal movement portion (53a) to the outside of the housing (2) when the support tray (10) is detached from the housing (2).

10. The air filtering apparatus according to claim 1, further comprising an air guide plate for guiding air from an air blow-out port (7a) of the air blowing fan (7) to the gas-liquid contact member (5), wherein the air guide plate comprises an extension portion (50a) extending in a flow direction of air blown out from the air blowing fan (7), and a guide portion (50b) that is connected to the extension portion (50a) and extends obliquely from the blow-out port (7a) toward the gas-liquid contact member.

11. The air filtering apparatus according to claim 10 further comprising a support plate (8) for isolating the gas-liquid contact member and the air blowing fan from each other, wherein the support plate (8) is provided with an opening (8a) through which the air blown out from the air blowing fan is passed.

12. The air filtering apparatus according to claim 10, wherein the inclination angle of the guide portion (50b) is adjustable with respect to the extension portion (50a).

13. The air filtering apparatus according to claim 10, wherein the length of the extension portion (50a) is adjustable.

14. The air filtering apparatus according to claim 1, wherein the controller contains a control board portion having electric parts and a heat sink for the electric parts, and the heat sink is disposed so as to penetrate through an opening (51) formed in the partition plate (51) and face a suction port (3) of the air blowing fan (7).

15. The air filtering apparatus according to claim 14, wherein the inside of the housing (2) is vertically partitioned by one partition plate (51), the suction port (3) of the air blowing fan (7) is substantially horizontally disposed, and air blown out from the air blowing fan to the gas-liquid contact member (5) is sucked from the suction port (3) that is formed in the housing and located at the lower side of the air blowing fan.

16. The air filtering apparatus according to claim 14, wherein the electrolytic water supply unit has electrodes for electrolyzing water to generate electrolytic water and the electric parts contain a part for controlling the electrodes of the electrolytic water supply unit.

17. The air filtering apparatus according to claim 14, wherein the electrolytic water generated in the electrolytic water supply unit is electrolytic water containing active oxygen species achieved by supplying current to the electrodes and electrolyzing tap water.

18. The air filtering apparatus according to claim 17, wherein the active oxygen species contain at least one of hypochlorous acid, ozone and hydrogen peroxide.

19. The air filtering apparatus according to claim 1, wherein the air filtering apparatus is a floor-mount type air filtering apparatus.

## Patentansprüche

1. Luftfilterungsvorrichtung, die ein Gehäuse (2), mindestens eine Trennplatte (51, 52) zum Aufteilen des Inneren des Gehäuses in mindestens zwei Kammern (60, 61, 62), ein Gas-Flüssigkeit-Kontaktelement (5), dem elektrolytisches Wasser zugeführt wird, eine Wasseraufnahmewanne (9) zum Aufnehmen des elektrolytischen Wassers, das aus dem Gas-Flüssigkeit-Kontaktelement herausfließt, einen Luftgebläseventilator (7) zum Blasen von Luft zu dem Gas-Flüssigkeit-Kontaktelement, so dass die Luft in Kontakt mit dem elektrolytischen Wasser, das dem Gas-Flüssigkeit-Kontaktelement zugeführt wird, gebracht wird, und eine Steuerung (52, 53) zum Steuern der Luftfilterungsvorrichtung umfasst, ***dadurch gekennzeichnet,* dass** sie weiter umfasst:
eine Zufuhreinheit (31) für elektrolytisches Wasser zum Erzeugen von elektrolytischem Wasser und Zuführen des erzeugten elektrolytischen Wassers zu dem Gas-Flüssigkeit-Kontaktelement;
eine Umwälzpumpe (13) zum Zuführen des elektrolytischen Wassers, das in der Wasseraufnahmewanne (9) aufgenommen wird, zu der Zufuhreinheit (31) für elektrolytisches Wasser, so dass das elektrolytische Wasser aufeinanderfolgend durch die Zufuhreinheit (31) für elektrolytisches Wasser, das Gas-Flüssigkeit-Kontaktelement (5), die Wasseraufnahmewanne (9) und die Umwälzpumpe (13) in dieser Reihenfolge zirkuliert, wobei das Gas-Flüssigkeit-Kontaktelement (5) und der Luftgebläseventilator (7) in einer der Kammern angeordnet sind und einer von der Steuerung und der Zufuhreinheit für elektrolytisches Wasser in der anderen Kammer der mindestens zwei Kammern angeordnet ist.

2. Luftfilterungsvorrichtung gemäß Anspruch 1, wobei ein Endteil (9A) der Wasseraufnahmewanne (9) durch eine Trennplatte (51) hindurch dringt, so dass sie sich zu der anderen Kammer (61) erstreckt.

3. Luftfilterungsvorrichtung gemäß Anspruch 2, wobei ein Abdeckungselement (72) zum Abdecken des Endbereichs (9A) der Wasseraufnahmewanne (9) in der anderen Kammer (61) vorgesehen ist.

4. Luftfilterungsvorrichtung gemäß Anspruch 2, wobei eine Antikorrosionsbeschichtung auf die Wasseraufnahmewanne (9) aufgebracht ist.

5. Luftfilterungsvorrichtung gemäß Anspruch 2, wobei ein Wasserrückführungsteil (71) an dem Endteil der Wasseraufnahmewanne vorgesehen ist.

6. Luftfilterungsvorrichtung gemäß Anspruch 2, wobei die Wasseraufnahmewanne (9) so angeordnet ist, dass sie von der anderen Kammer (61) zu der einen Kammer (60) abwärts geneigt ist.

7. Luftfilterungsvorrichtung gemäß Anspruch 1, wobei das elektrolytische Wasser eine aktive Sauerstoffspezies enthält, die durch das Elektrolysieren von Wasser erhalten wird, und die aktive Sauerstoffspezies mindestens eine von hypochloriger Säure, Ozon und Wasserstoffperoxid enthält.

8. Luftfilterungsvorrichtung gemäß Anspruch 1, wobei die Zufuhreinheit für elektrolytisches Wasser eine Trägerwanne (10) zum Aufnehmen von elektrolytischem Wasser, welches aus der Wasseraufnahmewanne (9) fließt, die unterhalb des Gas-Flüssigkeit-Kontaktelements (5) angeordnet ist und das betreffende elektrolytische Wasser speichert, enthält und die Umwälzpumpe (13) in die Trägerwanne eingeführt ist und das elektrolytische Wasser, das in der Trägerwanne gespeichert ist, hoch pumpt, wobei einer von einem vorstehenden Teil (50A) und einer Schienennut (53), in die der vorstehende Teil (50A) so eingepasst ist, dass er entlang der Schienennut (53) bewegbar ist, an mindestens einer der beiden Seitenflächen (10c, 10d) der Trägerwanne (10) vorgesehen ist, der andere des vorstehenden Teils und der Schienennut an mindestens einer der inneren Seitenfläche (2Z) des Gehäuses und der Seitenfläche (52c) der Trennplatte (52) vorgesehen ist, die innere Seitenfläche (2Z) und die Seitenfläche (52c), die den Seitenflächen (10c, 10d) der Trägerwanne (10) gegenüber liegen, und die Schienennut (53) so ausgelegt ist, dass der vorstehende Teil (50A) entlang der Schienennut (53) abwärts geführt ist, bis die Umwälzpumpe (13) an der Außenseite der Trägerwanne (10) angeordnet ist.

9. Luftfilterungsvorrichtung gemäß Anspruch 8, wobei die Schienennut (53) einen Vertikalbewegungsteil (53b) und einen Horizontalbewegungsteil (53a), der mit dem Vertikalbewegungsteil (53b) in gegenseitiger Verbindung steht, umfasst, die Trägerwanne (10) in einer von einer Abwärtsrichtung und einer schrägen Abwärtsrichtung bewegbar ist und entlang dem horizontalen Bewegungsteil (53a) horizontal bewegbar ist und von einem Ende des Horizontalbewegungsteils (53a) zur Außenseite des Gehäuses (2) abnehmbar ist, wenn die Trägerwanne (10) von dem Gehäuse (2) abgenommen ist.

10. Luftfilterungsvorrichtung gemäß Anspruch 1, die weiter eine Luftleitplatte zum Leiten von Luft von einem Luftausblasauslass (7a) des Luftgebläseventilators (7) zu dem Gas-Flüssigkeit-Kontaktelement (5) umfasst, wobei die Luftleitplatte einen Erstreckungsteil (50a) umfasst, der sich in einer Fließrichtung von Luft, die aus dem Luftgebläseventilator (7) herausgeblasen wird, erstreckt, und einen Leitteil (50b), der mit dem Erstreckungsteil (50a) verbunden ist und sich von dem Ausblasauslass (7a) in Richtung des Gas-Flüssigkeit-Kontaktelements schräg erstreckt.

11. Luftfilterungsvorrichtung gemäß Anspruch 10, die weiter eine Trägerplatte (8) zum Isolieren des Gas-Flüssigkeit-Kontaktelements und des Luftgebläseventilators voneinander umfasst, wobei die Trägerplatte (8) mit einer Öffnung (8a) versehen ist, durch welche die Luft, die aus dem Luftgebläseventilator herausgeblasen wird, hindurch geleitet wird.

12. Luftfilterungsvorrichtung gemäß Anspruch 10, wobei der Neigungswinkel des Leitteils (50b) hinsichtlich des Erstreckungsteils (50a) einstellbar ist.

13. Luftfilterungsvorrichtung gemäß Anspruch 10, wobei die Länge des Erstreckungsteils (50a) einstellbar ist.

14. Luftfilterungsvorrichtung gemäß Anspruch 1, wobei die Steuerung einen Steuertafelteil mit elektrischen Teilen und eine Wärmesenke für die elektrischen Teile enthält und die Wärmesenke so angeordnet ist, dass sie durch eine Öffnung (51), die in der Trennplatte (51) gebildet ist, hindurch dringt und einem Ansaugeinlass (3) des Luftgebläseventilators (7) gegenüber liegt.

15. Luftfilterungsvorrichtung gemäß Anspruch 14, wobei das Innere des Gehäuses (2) durch eine Trennplatte (51) vertikal unterteilt ist und der Ansaugeinlass (3) des Luftgebläseventilators (7) im Wesentlichen horizontal angeordnet ist und Luft, die aus dem Luftgebläseventilator zu dem Gas-Flüssigkeit-Kontaktelement (5) herausgeblasen wird, von dem Ansaugeinlass (3), der in dem Gehäuse gebildet ist und an der unteren Seite des Luftgebläseventilators angeordnet ist, angesaugt wird.

16. Luftfilterungsvorrichtung gemäß Anspruch 14, wobei die Zufuhreinheit für elektrolytisches Wasser Elektroden zum Elektrolysieren von Wasser aufweist, um elektrolytisches Wasser zu erzeugen, und die elektrischen Teile einen Teil zum Steuern der Elektroden der Zufuhreinheit für elektrolytisches Wasser enthalten.

17. Luftfilterungsvorrichtung gemäß Anspruch 14, wobei das elektrolytische Wasser, das in der Zufuhreinheit für elektrolytisches Wasser erzeugt wird, elektrolytisches Wasser ist, das eine aktive Sauerstoffspezies enthält, die durch das Zuführen von Strom zu den Elektroden und Elektrolysieren von Leitungswasser erhalten wird.

18. Luftfilterungsvorrichtung gemäß Anspruch 17, wobei die aktive Sauerstoffspezies mindestens eine von hypochloriger Säure, Ozon und Wasserstoffperoxid enthält.

19. Luftfilterungsvorrichtung gemäß Anspruch 1, wobei die Luftfilterungsvorrichtung eine Luftfilterungsvorrichtung vom Bodenbefestigungstyp ist.

## Revendications

1. Appareil de filtration de l'air comprenant un logement (2), au moins une plaque de séparation (51, 52) pour séparer l'intérieur du logement en au moins deux chambres (60, 61, 62), un organe de contact gaz-liquide (5) fourni avec de l'eau électrolytique, un plateau récepteur d'eau (9) pour recevoir l'eau électrolytique s'écoulant hors de l'organe de contact gaz-liquide, un ventilateur soufflant de l'air (7) pour souffler de l'air vers l'organe de contact gaz-liquide de sorte que l'air est mis en contact avec l'eau électrolytique fournie à l'organe de contact gaz-liquide, et un dispositif de commande (52, 53) pour commander l'appareil de filtration d'air, **caractérisé en ce qu'**il comprend en outre :
une unité d'alimentation en eau électrolytique (31) pour générer de l'eau électrolytique et fournir l'eau électrolytique générée à l'organe de contact gaz-liquide ;
une pompe de circulation (13) pour charger l'eau électrolytique reçue dans le plateau récepteur d'eau (9) à l'unité d'alimentation en eau électrolytique (31) de sorte que l'eau électrolytique circule successivement à travers l'unité d'alimentation en eau électrolytique (31), l'organe de contact gaz-liquide (5), le plateau récepteur d'eau (9) et la pompe de circulation (13) dans cet ordre, l'organe de contact gaz-liquide (5) et le ventilateur soufflant de l'air (7) étant disposés dans l'une des chambres, et l'un du dispositif de commande et de l'unité d'alimentation en eau électrolytique étant disposé dans l'autre chambre des au moins deux chambres.

2. Appareil de filtration de l'air selon la revendication 1, dans lequel une portion d'extrémité (9A) du plateau récepteur d'eau (9) pénètre à travers une plaque de séparation (51) de façon à s'étendre vers l'autre chambre (61).

3. Appareil de filtration de l'air selon la revendication 2, dans lequel un organe de couvercle (72) pour couvrir la portion d'extrémité (9A) du plateau récepteur d'eau (9) est prévu dans l'autre chambre (61).

4. Appareil de filtration de l'air selon la revendication 2, dans lequel un revêtement antirouille est appliqué sur le plateau récepteur d'eau (9).

5. Appareil de filtration de l'air selon la revendication 2, dans lequel une portion de renvoi de l'eau (71) est prévue sur la portion d'extrémité du plateau récepteur d'eau.

6. Appareil de filtration de l'air selon la revendication 2, dans lequel le plateau récepteur d'eau (9) est disposé de façon à s'incliner vers le bas à partir de l'autre chambre (61) vers la première chambre (60).

7. Appareil de filtration de l'air selon la revendication 1, dans lequel l'eau électrolytique contient une espèce d'oxygène actif obtenue par électrolyse de l'eau, et l'espèce d'oxygène actif contient au moins un élément parmi l'acide hypochloreux, l'ozone et le peroxyde d'hydrogène.

8. Appareil de filtration de l'air selon la revendication 1, dans lequel l'unité d'alimentation en eau électrolytique contient un plateau de support (10) pour recevoir de l'eau électrolytique s'écoulant à partir du plateau récepteur d'eau (9) situé sous l'organe de contact gaz-liquide (5) et stocker l'eau électrolytique concernée, et la pompe de circulation (13) est insérée dans le plateau de support et pompe l'eau électrolytique stockée dans le plateau de support, où un élément parmi une portion en saillie (50A) et une rainure de rail (53) dans laquelle est adaptée la portion en saillie (50A) de façon à être mobile le long de la rainure de rail (53) est prévu sur au moins l'une des deux surfaces latérales (10c, 10d) du plateau de support (10), l'autre élément parmi la portion en saillie et la rainure de rail étant prévu sur au moins l'une de la surface latérale interne (2Z) du logement et la surface latérale (52c) de la plaque de séparation (52), la surface latérale interne (2Z) et la surface latérale (52c) se trouvant face aux surfaces latérales (10c, 10d) du plateau de support (10), et la rainure de rail (53) étant conçue de sorte que la portion en saillie (50A) est guidée vers le bas le long de la rainure de rail (53) jusqu'à ce que la pompe de circulation (13) soit située à l'extérieur du plateau de support (10).

9. Appareil de filtration de l'air selon la revendication 8, dans lequel la rainure de rail (53) comprend une portion de mouvement vertical (53b) et une portion de mouvement horizontal (53a) en intercommunication avec la portion de mouvement vertical (53b), le plateau de support (10) étant mobile dans l'une d'une direction vers le bas et d'une direction vers le bas à l'oblique et mobile horizontalement le long de la portion de mouvement horizontal (53a) et détachable d'une extrémité de la portion de mouvement horizontal (53a) à l'extérieur du logement (2) lorsque le plateau de position (10) est détaché du logement (2).

10. Appareil de filtration de l'air selon la revendication 1, comprenant en outre une plaque de guidage de l'air pour guider l'air d'un orifice d'échappement de l'air (7a) du ventilateur soufflant de l'air (7) à l'organe de contact gaz-liquide (5), où la plaque de guidage de l'air comprend une portion d'extension (50a) s'étendant dans une direction d'écoulement de l'air soufflé par le ventilateur soufflant de l'air (7), et une portion de guidage (50b) qui est raccordée à la portion d'extension (50a) et s'étend à l'oblique de l'orifice d'échappement (7a) vers l'organe de contact gaz-liquide.

11. Appareil de filtration de l'air selon la revendication 10, comprenant en outre une plaque de support (8) pour isoler l'organe de contact gaz-liquide et le ventilateur soufflant de l'air l'un de l'autre, où la plaque de support (8) est munie d'une ouverture (8a) à travers laquelle passe l'air soufflé par le ventilateur soufflant de l'air.

12. Appareil de filtration de l'air selon la revendication 10, dans lequel l'angle d'inclinaison de la portion de guidage (50b) est réglable par rapport à la portion d'extension (50a).

13. Appareil de filtration de l'air selon la revendication 10, dans lequel la longueur de la portion d'extension (50a) est réglable.

14. Appareil de filtration de l'air selon la revendication 1, dans lequel le dispositif de commande contient une portion de tableau de commande ayant des pièces électriques et un dissipateur thermique pour les pièces électriques, et le dissipateur thermique est disposé de façon à pénétrer à travers l'ouverture (51) formée dans la plaque de séparation (51) et à faire face à un orifice d'aspiration (3) du ventilateur soufflant de l'air (7).

15. Appareil de filtration de l'air selon la revendication 14, dans lequel l'intérieur du logement (2) est séparé verticalement par une plaque de séparation (51), l'orifice d'aspiration (3) du ventilateur soufflant de l'air (7) est disposé sensiblement horizontalement, et l'air soufflé par le ventilateur soufflant de l'air vers l'organe de contact gaz-liquide (5) est aspiré par l'orifice d'aspiration (3) qui est formé dans le logement et situé au niveau du côté inférieur du ventilateur soufflant de l'air.

16. Appareil de filtration de l'air selon la revendication 14, dans lequel l'unité d'alimentation en eau électrolytique comporte des électrodes permettant d'électrolyser l'eau pour générer de l'eau électrolytique et les pièces électriques contiennent une pièce permettant de commander les électrodes de l'unité d'alimentation en eau électrolytique.

17. Appareil de filtration de l'air selon la revendication 14, dans lequel l'eau électrolytique générée dans l'unité d'alimentation en eau électrolytique est une eau électrolytique contenant une espèce d'oxygène actif obtenue par fourniture d'un courant aux électrodes et électrolyse d'eau du robinet.

18. Appareil de filtration de l'air selon la revendication 17, dans lequel l'espèce d'oxygène actif contient au moins un élément parmi l'acide hypochloreux, l'ozone et le peroxyde d'hydrogène.

19. Appareil de filtration de l'air selon la revendication 1, dans lequel l'appareil de filtration de l'air est un appareil de filtration de l'air de type à montage au sol.
